# DEMANDE DE BREVET EUROPEEN

(11) **EP 2 901 998 A1**
(43) Date de publication de la demande: **05.08.2015**
(21) Numéro de dépôt: 14447001.0
(22) Date de dépôt: 31.01.2014
(51) Int. Cl.: A61H 5/00

(54) **Panneau panoramique stéréoscopique de rééducation vestibulo-oculaire**

(71) Demandeur: Bauwens, Alain, 6600 Bastogne (BE)
(72) Inventeur: Bauwens, Alain, 6600 Bastogne (BE)

(57) **Abrégé**

Dispositif permettant d'améliorer un traitement pratiqué en orthoptie dans le but de soulager des patients souffrant de sensations vertigineuses et de symptômes associés, le dispositif comprenant un panneau panoramique stéréoscopique de rééducation vestibulo-oculaire, le panneau étant de fond blanc sur lequel est imprimé un décor visuel composé d'une trame régulière de points noirs et un quadrillage de lignes colorées.

## Description

Le panneau panoramique stéréoscopique de rééducation vestibulo-oculaire est un dispositif permettant d'améliorer un traitement pratiqué en orthoptie dans le but de soulager des patients souffrant de sensations vertigineuses et de symptômes associés.

### Caractéristiques:

- Le panneau doit avoir les dimensions suivantes:
   - hauteur: minimum 125 cm
   - largeur: minimum 210 cm
- Les points noirs doivent avoir une dimension qui évolue progressivement de 13 à 25 mm.
- Le quadrillage doit être formé de carrés de 6 cm decôtés, évoluant progressivement jusqu'à 11,5 cm de côté, et chacun des carré doit contenir 4 points noirs.
- Le support doit soutenir le panneau de manière à lui donner une forme incurvée selon un axe vertical et à créer un volume cylindrique partiel dont le rayon doit se situer entre 65 cm et 75 cm.
- Le support doit soutenir le panneau de manière à ce que son centre se positionne à hauteur des yeux du patient assis face face à lui, soit une hauteur approximative pouvant aller de 100 cm à 120 cm.

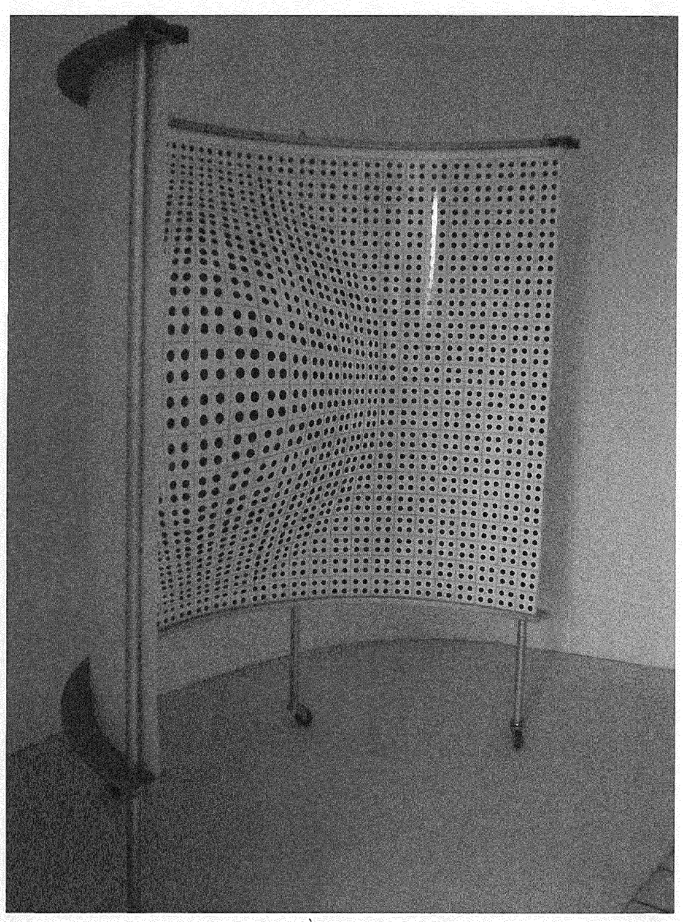

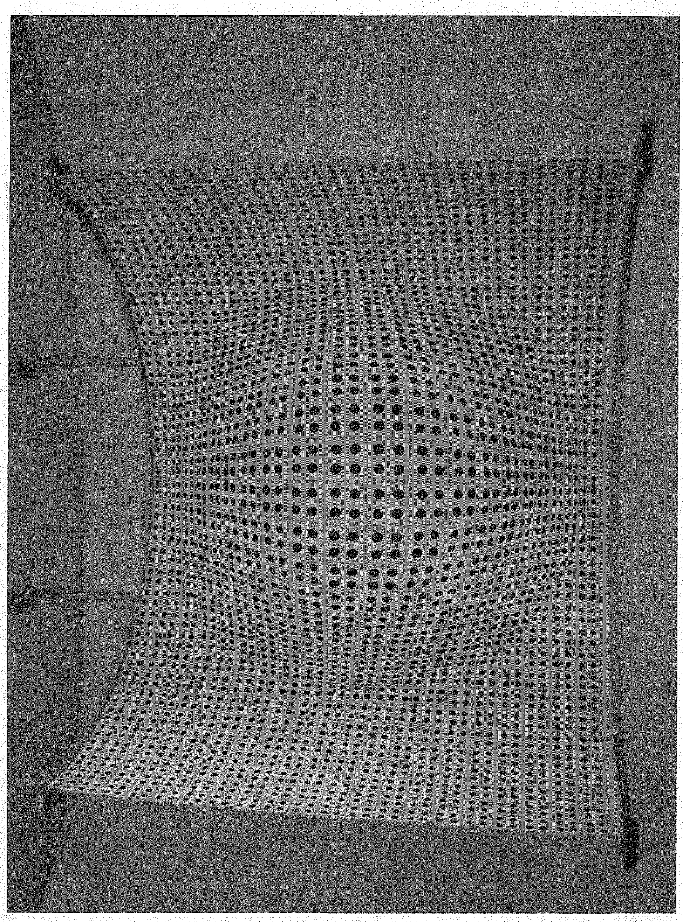

## Revendications

1. Le panneau panoramique stéréoscopique de rééducation vestibulo-oculaire est un dispositif permettant d'améliorer un traitement pratiqué en orthoptie dans le but de soulager des patients souffrant de sensations vertigineuses et de symptômes associés.
1. C'est un panneau de fond blanc sur lequel est imprimé un décor visuel composé d'une trame régulière de points noirs et un quadrillage de lignes colorées.
2. La particularité de ce décor visuel réside dans le fait qu'il est graphiquement déformé en son centre de façon à ce que l'ensemble homogène donne l'illusion optique d'être bombé vers l'avant.
3. Ce panneau est placé verticalement sur un support (fixe ou mobile) qui le rend incurvé selon un axe vertical et de façon à ce que le décor visuel soit présenté vers l'intérieur du volume créé.
